# FASCICULE DE BREVET EUROPEEN

(11) **EP 3 877 458 B1**
(45) Date de publication et mention de la délivrance du brevet: **31.12.2025**
(21) Numéro de dépôt: 19795244.3
(22) Date de dépôt: 05.11.2019
(51) Int. Cl.: C08J 11/10

(54) **PROCEDE DE PRODUCTION D'ACIDE TEREPHTALIQUE A L'ECHELLE INDUSTRIELLE**
VERFAHREN ZUR HERSTELLUNG VON TEREPHTHALSÄURE IM INDUSTRIELLEN MASSSTAB
PROCESS FOR PRODUCING TEREPHTHALIC ACID IN INDUSTRIAL SCALE

(30) Priorité: 06.11.2018 FR 1860220
(43) Date de publication de la demande: 15.09.2021
(73) Titulaire: Carbios, 63100 Clermont-Ferrand (FR)
(72) Inventeur: CHATEAU, Michel, 63100 Clermont-Ferrand (FR)
(74) Mandataire: Cabinet Becker et Associés
(86) Numéro de dépôt international: PCT/EP2019/080277
(87) Numéro de publication internationale: WO 2020/094661

(56) Documents cités:
- WO-A1-2017/198786
- CN-A- 108 554 342
- JP-A- 2004 290 130
- US-A1- 2005 261 465

## Description

### DOMAINE TECHNIQUE DE L'INVENTION

La présente invention concerne un procédé de production d'acide téréphtalique à l'échelle industrielle ou semi-industrielle par voie enzymatique à partir d'un polyester d'intérêt.

### ARRIERE PLAN TECHNOLOGIQUE

Les produits plastiques sont des matériaux durables et peu chers qui peuvent être utilisés pour la fabrication d'une grande variété de produits pour des applications diverses (emballages alimentaires, textiles d'habillement, etc.). En conséquence, la production de plastiques a drastiquement augmenté depuis les dernières décennies. La plupart d'entre eux sont utilisés pour des applications de courte durée, ce qui a pour effet d'entraîner une accumulation de déchets plastiques et une nécessité de traitement de ces derniers. Parmi les différents polymères constituants ces-dits plastiques, on retrouve notamment le polyéthylène téréphtalate (PET), un polyester aromatique produit à partir d'acide téréphtalique et d'éthylène glycol et qui est utilisé dans de nombreuses applications telles que les emballages alimentaires (bouteilles, flacons, pots, barquettes, poches), mais également dans la production de textiles pour l'habillement, la décoration (moquette), le linge de maison, etc.

Afin de répondre aux problématiques environnementales et économiques d'accumulation des déchets, des technologies de recyclage ou de valorisation énergétique ont été développées. Le procédé de recyclage mécanique reste aujourd'hui le plus utilisé, mais ce dernier présente de nombreux inconvénients. Sa mise en œuvre nécessite en effet de procéder à un tri sophistiqué et coûteux et conduit à la production de plastiques recyclés de qualité diminuée destinés à des applications de moindre valeur (baisse de masse moléculaire, présence incontrôlée d'additifs). En outre, ces plastiques recyclés ne sont pas compétitifs par rapport aux plastiques vierges issus du pétrole.

Récemment, des procédés innovants de recyclage enzymatique de produits plastiques ont été développés et notamment décrits dans les demandes de brevets WO 2014/079844, WO 2015/097104, WO 2015/173265 et WO 2017/198786. Contrairement aux procédés traditionnels de recyclage mécanique, ces procédés enzymatiques permettent, par dépolymérisation enzymatique du polymère contenu dans le plastique, de revenir aux principaux constituants (monomères) du polymère. Les monomères obtenus peuvent ensuite être purifiés et utilisés pour repolymériser de nouveaux polymères. Ces procédés enzymatiques permettent, via la spécificité des enzymes, d'éviter un tri couteux des plastiques, mais également de proposer un recyclage à l'infini conduisant à des polymères recyclés de qualité équivalente aux polymères issus du pétrole. Ces procédés permettent notamment, à partir de PET, de produire de l'acide téréphtalique et de l'éthylène glycol.

Une des problématiques associées à la production de monomères issus d'une dépolymérisation est l'étape de récupération desdits monomères. En effet, il est difficile de séparer les monomères sous forme solide tels que l'acide téréphtalique, du reste des déchets solides présents dans le réacteur, et notamment du polyester non encore dépolymérisé. Une telle étape de récupération est complexe et coûteuse, et la rend difficilement compatible avec une utilisation à l'échelle industrielle.

En travaillant sur ces problématiques, les Demandeurs ont mis au point un procédé enzymatique optimisé, permettant de produire à l'échelle industrielle de l'acide téréphtalique à partir de plastiques et/ou textiles contenant un polyester comprenant de l'acide téréphtalique, et notamment du PET.

### RESUME DE L'INVENTION

L'inventeur a développé un procédé de production d'acide téréphtalique à partir d'au moins un polyester comprenant de l'acide téréphtalique, et aboutissant à une production à forte concentration d'acide téréphtalique, permettant ainsi de répondre aux contraintes techniques et économiques d'une production à l'échelle industrielle. Plus précisément, l'inventeur a mis au point un procédé permettant d'introduire dans un réacteur une forte concentration en polyester, tout en conservant une vitesse de dépolymérisation dudit polyester compatible avec une viabilité industrielle. Particulièrement, l'inventeurs a identifié que la régulation du pH entre 6,5 et 9 dans un réacteur, sous agitation, permet de maintenir une part importante de l'acide téréphtalique produit sous forme soluble. La forte concentration en acide téréphtalique soluble est particulièrement avantageuse, puisqu'elle permet de simplifier l'étape de récupération de ces monomères et donc de diminuer les coûts de production.

En outre, le procédé développé par l'inventeur permet de maintenir des vitesses de dépolymérisation à l'intérieur du réacteur compatibles avec une mise en œuvre à l'échelle industrielle. A titre d'exemple, l'inventeur est parvenu à dépolymériser plus de 90% d'un polyester d'intérêt contenant de l'acide téréphtalique en seulement 24h, aboutissant à la récupération de plus de 90% de l'acide téréphtalique présent dans le polyester d'intérêt. Le procédé objet de l'invention peut être mis en œuvre sur n'importe quel déchet plastique contenant un polyester comprenant de l'acide téréphtalique. Les déchets plastiques peuvent être directement introduits dans le réacteur, sans tri sophistiqué ni prétraitement élaboré. De manière avantageuse, le procédé de l'invention peut être mis en œuvre pour la dépolymérisation et/ou le recyclage de plastiques. Le procédé de l'invention peut être mis en œuvre pour le recyclage de polyesters comprenant au moins une unité d'acide téréphtalique, principalement pour le recyclage de polyesters semi-aromatiques, notamment sélectionnés parmi le polyéthylène téréphtalate (PET), le polyéthylène téréphtalate glycosylé (PETG), le polyéthylène téréphtalate-co-isosorbide (PEIT), le polytriméthylène téréphtalate (PTT), le polybutylène adipate téréphtalate (PBAT), le polycyclohéxylènediméthylène téréphtalate (PCT) et le polybutylène téréphtalate (PBT).

L'invention a donc pour objet un procédé de production d'acide téréphtalique (AT) à partir d'au moins un polyester d'intérêt comprenant au moins une unité d'AT, comprenant une étape de dépolymérisation enzymatique du polyester selon laquelle ledit polyester est mis en contact avec au moins une enzyme apte à dépolymériser ledit polyester dans un réacteur sous agitation et une étape de récupération de sels d'AT sous forme solubilisée, caractérisé en ce que la quantité de polyester engagée dans le réacteur est supérieure ou égale à 11% en poids par rapport au poids total du milieu réactionnel initial, en ce que le pH est régulé entre 6,5 et 9 pendant l'étape de dépolymérisation, en ce que la température est régulée entre 35°C et 90°C pendant l'étape de dépolymérisation, en ce que l'étape de dépolymérisation dure entre 1h et 120h, et en ce que la concentration en AT dans la phase liquide du milieu réactionnel final est supérieure à 40kg/t .

Préférentiellement, l'étape de récupération des sels d'AT solubilisés comprend une étape de séparation de la phase liquide contenant les sels d'AT du reste du milieu réactionnel final. De manière préférée, le polyester d'intérêt est choisi parmi PTT, PBAT, PBT, PET, PETG, PEIT, PCT. Plus préférentiellement le polyester d'intérêt est le PET.

Avantageusement, le polyester d'intérêt est introduit dans le réacteur sous forme de poudre et/ou de granules, notamment sous forme de poudre et/ou de granules de granulométrie inférieure à 2 mm, préférentiellement inférieure à 1 mm.

De manière avantageuse, l'étape de dépolymérisation du procédé de l'invention dure au plus 150h, et plus préférentiellement au plus 48h. Par ailleurs, le procédé selon l'invention peut être mis en œuvre dans des réacteurs de taille industrielle, et notamment des réacteurs présentant un volume utile de plusieurs litres, plusieurs dizaines de litres, plusieurs centaines de litres. Préférentiellement, le pH est régulé pendant l'étape de dépolymérisation par l'ajout dans le milieu réactionnel d'une solution basique concentrée à au moins 10% +/- 1%.

Avantageusement, les sels d'AT récupérés peuvent être réutilisés sous forme d'AT, notamment pour la production de nouveaux polyesters.

L'invention a également pour objet l'utilisation d'un réacteur présentant un volume supérieur à 1000 L, pour la mise en œuvre des procédés décrits ci-dessus.

### DESCRIPTION DETAILLEE DE L'INVENTION

### Définitions

Dans le contexte de l'invention l'expression « matériau plastique » désigne les produits plastiques (tels que des feuilles, barquettes, films, tubes, blocs, fibres, tissus, etc.) et les compositions plastiques utilisées pour réaliser les produits plastiques. De manière préférentielle, le matériau plastique est composé de polymères amorphes et/ou semi-cristallins. Le matériau plastique peut contenir en plus du ou des polymères, des substances additionnelles ou des additifs, tels que des plastifiants, des charges minérales ou organiques, des colorants, etc. Ainsi, dans le contexte de l'invention, le matériau plastique fait référence à tout produit plastique et/ou composition plastique comprenant au moins un polymère sous forme semi-cristalline et/ou amorphe, plus particulièrement au moins un polyester.

Les produits plastiques désignent notamment les produits plastiques manufacturés, tels que les emballages rigides ou souples (films, bouteilles, barquettes), films agricoles, sacs, objets jetables, textiles, tissus, non-tissés, revêtements de sols, les déchets plastiques ou déchets fibres, etc.

Le terme « polymère » fait référence à un composé chimique dont la structure est constituée de multiples unités répétées (i.e. « monomères ») liées par des liaisons covalentes chimiques. Dans le contexte de l'invention, le terme « polymère » fait plus précisément référence à de tels composés chimiques entrant dans la composition de matériaux plastiques.

Le terme « polyester » fait référence à un polymère qui contient un groupe fonctionnel ester dans la chaîne principale de sa structure. Le groupe fonctionnel ester est caractérisé par une liaison entre un carbone et trois autres atomes : une liaison unique avec un autre atome de carbone, une double liaison avec un oxygène et une liaison simple avec un autre atome d'oxygène. L'oxygène lié au carbone par une liaison simple est lui-même lié à un autre carbone par une liaison simple. Les polyesters peuvent être constitués d'un seul type de monomères (i.e. homopolymère) ou d'au moins deux monomères différents (i.e. copolymère). Les polyesters peuvent être aromatiques, aliphatiques ou semi-aromatiques. A titre d'exemple, le polyéthylène téréphtalate est un copolymère semi-aromatique composé de deux monomères, l'acide téréphtalique et l'éthylène glycol. Dans le contexte de l'invention, le « polyester d'intérêt » désigne un polyester comprenant au moins une unité d'acide téréphtalique en tant que monomère.

Dans le contexte de l'invention, le terme « polymères semi-cristallins » fait référence à des polymères partiellement cristallins, dans lesquels des régions cristallines et amorphes coexistent. Le degré de cristallinité d'un polymère semi-cristallin peut être estimé par diverses méthodes analytiques, et est généralement compris entre 10% et 90%. Un polymère avec un degré de cristallinité inférieur à 10% peut être considéré comme amorphe. Dans la présente demande, la cristallinité est mesurée par analyse calorimétrique différentielle à balayage (DSC). La diffraction aux rayons X peut aussi être utilisée pour la mesure du degré de cristallinité.

Le terme « dépolymérisation », en relation avec un polymère ou un matériau plastique contenant un polymère, fait référence à un procédé par lequel un polymère ou au moins un polymère dudit matériau plastique est dépolymérisé en molécules plus petites, telles que des monomères et/ou des oligomères.

Tels qu'utilisés dans la présente demande, les termes « solubilisé » ou « sous forme solubilisée » désignent un composé dissous dans un liquide, à l'inverse des formes solides non dissoutes.

Le terme « acide téréphtalique » ou « AT » désigne la molécule d'acide téréphtalique seule c'est-à-dire C₈H₆O₄, correspondant à l'acide téréphtalique sous sa forme acide. Les termes « sels d'acide téréphtalique », « sels de téréphtalate » ou « sels d'AT » désignent un composé comprenant une molécule d'acide téréphtalique associée avec un/des cations tel que le sodium, potassium, ammonium. Dans le cadre de l'invention, les sels d'AT peuvent comprendre le disodium de téréphtalate C₈H₄Na₂O₄, le dipotassium de téréphtalate C₈H₄K₂O₄, le diammonium de téréphtalate C₈H₁₂N₂O₄, le monosodium de téréphtalate C₈H₅NaO₄, le monopotassium de téréphtalate de C₈H₅KO₄ et/ou le monoammonium de téréphtalate C₈H₁₀NO₄.

Selon l'invention, la concentration en acide téréphtalique dans la phase liquide du milieu réactionnel final correspond à la quantité d'AT mesurée à la fin de l'étape de dépolymérisation, quelle que soit sa forme, c'est-à-dire l'AT sous forme solubilisée ou non solubilisée, y compris sous forme de sel. La concentration en acide téréphtalique peut-être déterminée par tout moyen connu de l'homme de l'art, notamment par HPLC.

Dans le contexte de l'invention, « quantité engagée » désigne la quantité d'un composé, par exemple la quantité de polyester d'intérêt ou la quantité d'enzyme, introduite dans le réacteur au début (temps t=0) de l'étape de dépolymérisation du polyester. La quantité engagée de polyester, et notamment de PET, désigne la quantité en ce polyester, indépendamment des autres composés pouvant être présents dans le matériau plastique. Ainsi, dans le cas où le polyester est contenu dans un déchet plastique, la quantité engagée dudit polyester est différente de la quantité engagée en déchet plastique, ledit déchet plastique pouvant contenir d'autres composés en plus dudit polyester.

On entend par « milieu réactionnel », l'ensemble de la matière (incluant notamment du liquide, des enzymes, le polyester d'intérêt et/ou les monomères issus de la dépolymérisation dudit polyester) présent dans le réacteur pendant l'étape de dépolymérisation, i.e. le contenu du réacteur. On entend respectivement par « milieu réactionnel initial » et « milieu réactionnel final », le milieu réactionnel au début et à la fin de l'étape de dépolymérisation. Dans le cadre de l'invention, le volume total du réacteur est avantageusement supérieur d'au moins 10% au volume du milieu réactionnel final.

On entend par « phase liquide du milieu réactionnel final », le milieu réactionnel obtenu à l'issue de l'étape de dépolymérisation, débarrassé des particules solides et/ou en suspension.

Ladite phase liquide inclut le liquide et l'ensemble des composés dissous dans ce liquide (y compris les enzymes, monomères, sels, etc.). Cette phase liquide peut être obtenue par séparation d'avec la phase solide du milieu réactionnel, en utilisant des techniques classiques connues de l'homme de l'art, telles que la filtration, la centrifugation, etc. Dans le cadre de l'invention, la phase liquide est notamment dépourvue de polyester résiduel, c'est-à-dire non dégradé à la fin de l'étape de dépolymérisation.

### Procédé de dépolymérisation

Le procédé de production d'acide téréphtalique selon l'invention repose sur une dépolymérisation enzymatique d'au moins un polyester d'intérêt contenant dans ses constituants au moins une unité d'acide téréphtalique, par mise en contact dudit polyester d'intérêt avec au moins une enzyme apte à dépolymériser ledit polyester. Plus particulièrement, l'inventeur a mis au point un procédé permettant de produire des quantités importantes d'acide téréphtalique sous une forme facilement purifiable, dans un temps de réaction relativement faible. En effet, l'inventeur a découvert, de manière inattendue, qu'il est possible d'introduire des charges importantes de polyester d'intérêt et au moins une enzyme apte à le dépolymériser dans un réacteur, sous agitation et en maintenant un pH entre 6,5 et 9, et d'obtenir un taux de dépolymérisation particulièrement important aboutissant à des concentrations en acide téréphtalique supérieures à 40kg/t dans la phase liquide du milieu réactionnel dans un temps parfaitement acceptable à l'échelle industrielle et semi-industrielle. Le procédé selon l'invention permet en outre d'obtenir de l'acide téréphtalique sous forme solubilisée i.e. sous forme de sels d'AT, ce qui permet de le purifier aisément, rendant le procédé selon l'invention particulièrement avantageux à l'échelle industrielle.

Le procédé de production d'acide téréphtalique (AT) selon l'invention comprend ainsi
- une étape de dépolymérisation du polyester d'intérêt selon laquelle ledit polyester est mis en contact avec au moins une enzyme apte à dépolymériser ledit polyester dans un réacteur sous agitation, et
- une étape de récupération de sels d'AT sous forme solubilisée,
caractérisé en ce que le réacteur contient, au début de l'étape de dépolymérisation, une quantité de polyester engagée supérieure ou égale à 11% en poids par rapport au poids total du milieu réactionnel initial, en ce que le pH est régulé entre 6,5 et 9 pendant l'étape de dépolymérisation, en ce que la température est régulée entre 35°C et 90°C pendant l'étape de dépolymérisation, en ce que l'étape de dépolymérisation dure entre 1h et 120h, et en ce que la concentration en AT dans le réacteur à la fin de l'étape de dépolymérisation est supérieure à 40kg/t dans la phase liquide du milieu réactionnel final.

Avantageusement, la typologie des sels d'acide téréphtalique obtenus est liée à la base utilisée pour réguler le pH. Préférentiellement, les sels de téréphtalate produits lors de l'étape de dépolymérisation sont sous forme de téréphtalate de sodium, de téréphtalate de potassium et/ou de téréphtalate d'ammonium.

Selon l'invention, avec un pH régulé supérieur ou égal à 6.5, à l'issue de l'étape de dépolymérisation, on récupère des sels d'AT sous forme solubilisée dans la phase liquide du milieu réactionnel.

Dans un mode de réalisation particulier, le procédé de l'invention est mis en œuvre à l'échelle industrielle et semi-industrielle. Il est ainsi possible d'utiliser un réacteur dont le volume est supérieur à 1000L, 10 000 L, 100 000 L, 400 000 L. Le procédé est mis en œuvre dans un réacteur dont le volume est supérieur à 1000L.

### Contenu du réacteur

Le milieu réactionnel initial dans le réacteur comprend au moins le polyester d'intérêt, éventuellement contenu dans un matériau plastique et notamment dans un produit plastique ou un déchet plastique, l'enzyme dégradant ledit polyester et un liquide. Au fur et à mesure de l'étape de dépolymérisation, le milieu réactionnel s'enrichit en monomères et notamment en sels d'AT et la quantité de polyester d'intérêt diminue.

De manière préférée, le liquide dans le réacteur comprend un solvant aqueux, préférentiellement de l'eau. Dans un cas préféré, le liquide contenu dans le réacteur est dépourvu de solvant non aqueux, et notamment dépourvu de solvant organique. Dans un mode de réalisation, le liquide dans le réacteur ne comprend que de l'eau.

Selon l'invention, le polyester d'intérêt comprend au moins une unité d'acide téréphtalique en tant que monomère. Avantageusement, le polyester d'intérêt est choisi parmi le polytriméthylène téréphtalate (PTT), le polybutylène adipate téréphtalate (PBAT), le polybutylène téréphtalate (PBT), le polyéthylène téréphtalate (PET), le poly(éthylène téréphtalate-co-isosorbide) PEIT, le polycyclohéxylènediméthylène téréphtalate (PCT) et/ou des copolymères de ces derniers. Préférentiellement, le polyester d'intérêt est du PET. Dans un cas particulier, le polyester d'intérêt est choisi parmi les polyesters modifiés, préférentiellement le polyester d'intérêt est du PET modifié, tel que le PET glycosylé (PETG).

La quantité engagée de polyester d'intérêt dans le réacteur est supérieure ou égale à 11% en poids par rapport au poids total du milieu réactionnel initial, préférentiellement supérieure ou égale à 15%, préférentiellement supérieure ou égale à 20%. Particulièrement, la quantité engagée de polyester d'intérêt dans le réacteur est inférieure à 60% en poids par rapport au poids total du milieu réactionnel initial, préférentiellement inférieure à 50%. Dans un autre cas particulier, la quantité engagée de polyester dans le réacteur est comprise entre 15% et 25% en poids par rapport au poids total du milieu réactionnel initial, préférentiellement 20%, +/- 2%. Dans un autre cas particulier la quantité engagée de polyester dans le réacteur est comprise entre 11% et 20% en poids par rapport au poids total du milieu réactionnel initial, préférentiellement 15%, +/- 2%. Dans un mode de réalisation, la quantité engagée de polyester d'intérêt dans le réacteur est comprise entre 11 % et 60% en poids par rapport au poids total du milieu réactionnel initial, préférentiellement entre 15% et 50%, plus préférentiellement entre 15% et 40%, entre 15% et 30%, entre 15% et 25%, entre 20% et 30%, entre 20% et 25%. Dans le cas où plusieurs polyesters contenant au moins une unité d'acide téréphtalique sont utilisés dans le réacteur, la quantité de polyester engagée désigne les quantités cumulées en chacun des polyesters.

Selon l'invention, le polyester d'intérêt est un polyester amorphe et/ou semi-cristallin. De manière préférée, le polyester d'intérêt a un degré de cristallinité inférieur à 30%, préférentiellement inférieur à 25%, plus préférentiellement inférieur à 20%. Particulièrement le polyester d'intérêt à un degré de cristallinité inférieur à 30% +/- 10%, préférentiellement inférieur à 25% +/- 10%, plus préférentiellement inférieur à 20% +/- 10%. Dans un autre cas préféré, le polyester d'intérêt est un polyester amorphe. Selon l'invention, il est possible de procéder à une étape d'amorphisation du polyester d'intérêt avant l'étape de dépolymérisation par tout moyen connu de l'homme de l'art. Une telle étape d'amorphisation est notamment décrite dans la demande WO 2017/198786. Dans un mode de réalisation particulier, le polyester d'intérêt ou le matériau plastique contenant le polyester d'intérêt engagé dans le réacteur est sous forme de granules ou de micro-granules d'une taille inférieure à 5 mm, préférentiellement de granulométrie inférieure à 3 mm, plus préférentiellement de granulométrie inférieure à 2 mm. Selon l'invention, il est possible de procéder à une étape de pré-traitement du polyester d'intérêt, et notamment à une étape de broyage du polyester d'intérêt, ou du matériau plastique contenant le polyester d'intérêt avant l'étape de dépolymérisation du polyester. Dans un mode préféré, le polyester d'intérêt ou le matériau plastique contenant le polyester d'intérêt est réduit sous forme de poudre par tout moyen approprié connu de l'homme de l'art. Dans ce cas particulier, le polyester d'intérêt, ou le matériau plastique contenant le polyester d'intérêt, est avantageusement micronisé, de manière à être transformé sous forme de poudre.

Dans un mode de réalisation particulier, le procédé de production comprend une étape d'amorphisation du polyester d'intérêt, suivie d'une étape de broyage et/ou de micronisation du polyester d'intérêt ou du matériau plastique contenant le polyester d'intérêt avant l'étape de dépolymérisation du polyester.

Dans un mode de mise en œuvre particulier, le polyester d'intérêt ou le matériau plastique contenant le polyester d'intérêt engagé dans le réacteur est sous forme de poudre et/ou de granules de granulométrie moyenne (d50) inférieure à 2 mm, préférentiellement de granulométrie inférieure à 1 mm. Dans un autre mode de réalisation le polyester d'intérêt ou le matériau plastique contenant le polyester d'intérêt engagé dans le réacteur est sous forme de poudre de granulométrie moyenne (d50) inférieure à 500µm.

Préférentiellement, le polyester d'intérêt a un degré de cristallinité inférieur à 25% +/- 10%, et est engagé dans le réacteur sous forme de poudre et/ou de granules d'une taille inférieure à 2 mm, préférentiellement inférieure à 1 mm. Selon l'invention, il est possible de charger le réacteur directement en polyester d'intérêt, ou en matériaux plastiques contenant au moins le polyester d'intérêt.

Selon l'invention, le(s) matériau(x) plastique(s) engagé(s) dans le réacteur peuvent contenir un mélange de plusieurs polymères et notamment de plusieurs polyesters. Dans un mode de réalisation particulier, le procédé de dépolymérisation selon l'invention est mis en œuvre avec un matériau plastique comprenant au moins du PET. Dans un mode préféré, le PET représente au moins 80% en poids par rapport au poids total dudit matériau plastique, préférentiellement au moins 85%, 90%, 95%. Dans un mode de mise en œuvre particulier, le matériau plastique comprend un mélange de PET et d'acide polylactique (PLA), un mélange de PET et polyéthylène (PE), un mélange de PET et polytriméthylène téréphtalate (PTT), un mélange de PET et de polyamide (PA), ou un mélange de PET et de coton. Avantageusement, les matériaux plastiques engagés dans le réacteur sont des déchets plastiques ou déchets fibres. Ces déchets peuvent être issus des filières de collecte destinées au recyclage, mais également être des déchets de la filière de production ou de la filière de recyclage, et peuvent ainsi contenir d'autres composés que les déchets plastiques. Cela implique que le polyester d'intérêt peut être engagé dans le réacteur en combinaison d'autres éléments présents dans ces flux (tel que papier, carton, aluminium, colle etc...). Dans un mode de mise en œuvre particulier, le réacteur est chargé en plusieurs matériaux plastiques contenant au moins le polyester d'intérêt, préférentiellement au moins du PET, plus préférentiellement contenant au moins 80% de PET. Dans un autre mode de mise en œuvre particulier, le matériau plastique est sélectionné parmi des fibres et/ou des déchets fibres et/ou textiles et le PET représente au moins 60% en poids par rapport au poids total dudit matériau plastique, préférentiellement au moins 65%, 70%, 75%,80%, 85%, 90%, 95%.

Avantageusement, l'enzyme dégradant le polyester d'intérêt est sélectionnée parmi les cutinases, les lipases et les estérases dégradant ledit polyester. Particulièrement, ladite enzyme est sélectionnée parmi les estérases dégradant ledit polyester d'intérêt. Dans un mode de réalisation particulier, ledit polyester est du PET et l'enzyme est une cutinase dégradant le PET. Plus particulièrement, l'enzyme est une cutinase préférablement issue de *Thermobifida cellulosityca, Thermobifida halotolerans, Thermobifida fusca, Thermobifida alba, Bacillus subtilis, Fusarium solani pisi, Humicola insolens* (telle que celle référencée A0A075B5G4 dans la base Uniprot), *Sirococcus conigenus, Pseudomonas mendocina* et *Thielavia terrestris* ou un variant de celles-ci. Dans un autre cas, la cutinase est sélectionnée parmi les cutinases issues de banques métagénomiques telle que la LC-Cutinase décrite dans Sulaiman et al., 2012 ou des variants de cette dernière. Dans un autre cas, l'enzyme est une lipase, préférablement issue de Ideonella sakaiensis*.* Dans un cas alternatif, l'enzyme est sélectionnée parmi les enzymes commerciales telle que Novozym 51032 ou des variants de ces enzymes. Il est bien entendu possible de charger le réacteur avec plusieurs enzymes, et notamment au moins deux enzymes parmi celles évoquées ci-dessus.

Dans un cas particulier, l'enzyme (ou les enzymes) est sélectionnée parmi les enzymes ayant une séquence en acides aminés présentant au moins 75% d'identité avec SEQ ID N°1 et/ou avec SEQ ID N°2 et/ou avec SEQ ID N°3 et/ou avec SEQ ID N°4 et/ou SEQ ID N°5, et ayant une activité de dépolymérisation d'un polyester comprenant au moins une unité d'acide téréphtalique. Dans un cas particulier, l'enzyme est sélectionnée parmi les enzymes ayant une séquence en acides aminés présentant au moins 75% d'identité avec SEQ ID N°1, et une activité de dépolymérisation du PET.

Dans un mode particulier l'enzyme est apte à dépolymériser le polymère jusqu'aux oligomères, dans ce cas-là elle est avantageusement associée à une enzyme apte à dépolymériser lesdits oligomères en monomères. Dans un exemple particulier, les deux enzymes sont alors sélectionnées parmi les enzymes ayant une séquence en acides aminés présentant au moins 75% d'identité avec SEQ ID N°4 et/ou SEQ ID N°5.

L'inventeur a identifié que le procédé de l'invention était particulièrement adapté dans le cas particulier où l'enzyme sélectionnée a une séquence en acides aminés présentant au moins 90% d'identité avec SEQ ID N°1 et le polyester d'intérêt est préférentiellement sélectionné parmi le PET et/ou le PBAT. C'est particulièrement le cas des enzymes ayant la séquence en acides aminés de SEQ ID N°1 ou SEQ ID N°2. Contrairement aux autres enzymes connues pour dépolymériser les polyesters, ces enzymes subissent une inhibition limitée de leur activité par les monomères produits dans les conditions du procédé de l'invention.

C'est donc un objet de l'invention de proposer un procédé de production d'acide téréphtalique tel que décrit ci-dessus et caractérisé en ce que l'enzyme apte à dépolymériser ledit polyester est choisie parmi les enzymes ayant une séquence en acides aminés présentant au moins 90% d'identité avec SEQ ID N°1 et une activité de dépolymérisation d'un polyester comprenant au moins une unité d'acide téréphtalique et plus particulièrement une activité de dépolymérisation du PET.

Préférentiellement, le procédé de production d'acide téréphtalique selon l'invention est mis en œuvre en utilisant du PET et au moins une enzyme apte à dépolymériser ledit PET sélectionnée parmi les cutinases, telles que décrites ci-dessus.

Selon l'invention, la quantité d'enzyme dégradant le polyester d'intérêt engagée dans le réacteur est avantageusement suffisante pour permettre une dépolymérisation totale ou quasi-totale dudit polyester (i.e., à hauteur au moins de 80% en poids par rapport au poids dudit polyester engagée) dans des temps de réaction compatibles avec une mise en œuvre à l'échelle industrielle. Dans un mode de réalisation, le ratio en poids quantité d'enzyme engagée sur quantité de polyester engagée est compris entre 0,01/1000 et 3/1000. Préférentiellement le ratio quantité d'enzyme engagée sur quantité de polyester engagée est compris entre 0,5/1000 et 2,5/1000, plus préférentiellement entre 1/1000 et 2/1000. Dans un cas particulier, la quantité d'enzyme engagée est supérieure ou égale à la quantité d'enzyme nécessaire pour atteindre une concentration saturante en enzyme, i.e. une concentration au-delà de laquelle la vitesse de réaction n'est pas améliorée par l'ajout d'enzyme. Dans un cas particulier, l'enzyme peut être engagée sous la forme d'une composition comprenant en plus de l'enzyme des excipients, pouvant être sélectionnés parmi les tampons communément utilisés en biochimie, des conservateurs, et/ou des agents stabilisants. La quantité d'enzyme désigne alors avantageusement la quantité d'enzyme exempte de tout excipient.

Selon l'invention, le contenu du réacteur est maintenu sous agitation pendant l'étape de dépolymérisation. La vitesse de l'agitation est régulée par l'homme de l'art de manière à être suffisante pour permettre une suspension du matériau plastique/polyester engagé dans le réacteur, une homogénéité de la température et une précision de la régulation du pH. Notamment, l'agitation est maintenue à une vitesse comprise entre 50 et 500 rpm, par exemple 80 rpm, 100 rpm, 150 rpm, 200 rpm, 250 rpm, 300 rpm, 350 rpm, 400 rpm, 450 rpm, 500 rpm.

Dans un mode particulier, pour un réacteur d'un volume supérieur à 1000L, l'agitation est supérieure ou égale à 300 rpm. Dans un mode particulier, pour un réacteur d'un volume supérieur à 1000L, l'agitation est supérieure ou égale à 100 rpm.

La dépolymérisation du polyester d'intérêt produit des monomères acides susceptibles d'induire une diminution du pH du contenu du réacteur. Une addition de base peut être utilisée de manière à neutraliser l'acide produit et réguler le pH. Dans le cas de la présente invention, le pH peut notamment être régulé par l'ajout de toutes bases connues de l'homme de l'art. Particulièrement, le pH est régulé par l'ajout d'une base sélectionnée parmi l'hydroxyde de sodium (NaOH), hydroxyde de potassium (KOH) et/ou l'ammoniaque (NH4OH). Dans le cas d'une régulation du pH par l'ajout de base, l'AT produit va ainsi s'associer avec la/les base(s) utilisée(s) de manière à former des sels d'AT dont la solubilité est augmentée par rapport à l'AT. Avantageusement, le pH est régulé pendant l'étape de dépolymérisation par l'ajout dans le milieu réactionnel d'une solution basique concentrée à au moins 10% +/- 1%, en poids de base par rapport au poids total de la solution basique (comprenant essentiellement la base et de l'eau). Préférentiellement, la solution basique est concentrée à au moins 15% +/- 1% et au plus 50% +/-1%, plus préférentiellement au moins 20% +/- 1%. Dans un cas particulier, la solution basique est concentrée entre 20% et 50%, +/- 1%, plus préférentiellement entre 20% et 30%, +/- 1%, encore plus préférentiellement entre 20% et 25%, +/- 1%. Préférentiellement, la base est sélectionnée parmi l'hydroxyde de sodium (NaOH) et l'hydroxyde de potassium (KOH) et la solution basique est concentrée au moins à 15% et au plus 50%.

Le pH est ainsi régulé pour être maintenu entre 6,5 et 9, afin que l'acide téréphtalique produit soit majoritairement sous forme de sels d'AT solubilisés et/ou de façon à être au pH optimum de l'enzyme. Dans un mode de mise en œuvre particulier, le pH est régulé entre 6,5 et 8,5 pendant l'étape de dépolymérisation, préférentiellement entre 7 et 8. Dans un autre cas particulier, le pH est régulé entre 7,5 et 8,5. Préférentiellement le pH est régulé à 8 +/- 0,2.

La température au sein du réacteur et donc dans le milieu réactionnel, est régulée entre 35°C et 90°C pendant l'étape de dépolymérisation, préférentiellement entre 45°C et 80°C. Dans un mode préféré de l'invention, la température est régulée entre 55°C et 80°C, plus préférentiellement entre 60°C et 80°C. Dans un mode particulier, la température est régulée entre 60°C et 66°C.

Dans un cas particulier, le polyester d'intérêt a une température de transition vitreuse (Tg) supérieure à 30°C et la température au sein du réacteur est régulée à une température inférieure ou égale à la Tg du polyester d'intérêt. De manière alternative ou additionnelle, la température est régulée à la température optimale de l'enzyme utilisée. Dans un mode de réalisation particulier, le polyester d'intérêt est du PET dont la Tg est d'environ 70°C +/- 5°C, et la température au sein du réacteur est maintenue à 60°C +/- 5°C.

Selon l'invention, l'étape de dépolymérisation est conduite pendant un temps de réaction d'au plus 150h. Le temps de réaction dépend entre autres du couple polyester d'intérêt / enzyme de dépolymérisation et du taux de dépolymérisation du polyester souhaité. L'homme du métier saura adapter le temps de réaction de l'étape de dépolymérisation en fonction des critères précédemment cités. L 'étape de dépolymérisation dure entre 1h et 120h, entre 1h et 100h, entre 1h et 72h, entre 1h et 48h, entre 1h et 36h, entre 1h et 24h, entre 1h et 12h, entre 1h et 10h, entre 1h et 6h. Dans un mode préféré, le temps de l'étape de dépolymérisation est inférieur à 24h.

Dans un mode préféré, le temps de réaction ci-dessus permet d'atteindre une dépolymérisation du polyester d'intérêt à hauteur d'au moins 80%, préférentiellement d'au moins 85%, 90%, 95%. Préférentiellement, la dépolymérisation est conduite jusqu'aux monomères, c'est-à-dire qu'une dépolymérisation à hauteur de 80% conduit à une production à hauteur de 80% de monomères (et pas ou quasiment pas d'oligomères).

Dans un mode particulier, le procédé de production d'AT est mis en œuvre à partir de matériaux plastiques comprenant du PET et une enzyme dont la séquence en acides aminés comprend au moins la SEQ ID N°1, ledit procédé permettant une dépolymérisation d'au moins 80% du PET dans un temps inférieur à 72h, préférentiellement une dépolymérisation d'au moins 90% du PET est obtenu dans un temps inférieur à 72h. Dans un autre mode préféré, ledit procédé permet une dépolymérisation d'au moins 80% du PET dans un temps inférieur à 48h.

Selon l'invention, l'étape de dépolymérisation peut être réalisée dans tout réacteur habituellement utilisé dans l'industrie chimique ou en production biologique, comme par exemple un fermenteur. De manière générale, selon l'invention, l'étape de dépolymérisation peut être réalisée dans toute cuve ou tout réacteur dont la température et le pH peuvent être régulés et muni de moyens d'agitation pour homogénéiser le milieu.

### Production d'acide téréphtalique

Le procédé selon l'invention permet de produire de fortes concentrations d'acide téréphtalique en des temps de réaction parfaitement compatibles avec les contraintes industrielles.

Plus particulièrement, le procédé selon l'invention permet d'obtenir à l'issue de l'étape de dépolymérisation une concentration en acide téréphtalique d'au moins 40kg/t par rapport au poids total de la phase liquide du milieu réactionnel final. Avantageusement on considère que l'étape de dépolymérisation est arrivée à son terme lorsque le taux de dépolymérisation du polyester d'intérêt atteint au moins 80%, préférentiellement au moins 90%. Dans un mode particulier, l'étape de dépolymérisation peut être stoppée par l'homme du métier quand les rendements atteints sont compatibles avec des contraintes industrielles, i.e. quand le taux de dépolymérisation du polyester d'intérêt atteint 80% +/-10%, préférentiellement 90% +/-5%. Ainsi, dans le contexte de l'invention, l'issue ou fin de l'étape de dépolymérisation correspond au moment où la dépolymérisation du polyester est stoppée, et/ou au moment où le taux de dépolymérisation du polyester d'intérêt atteint au moins 80%, préférentiellement au moins 90% et/ou au moment où l'étape de récupération des sels d'AT commence.

Préférentiellement, la concentration en acide téréphtalique obtenue à partir du polyester d'intérêt à l'issue de l'étape de dépolymérisation est supérieure à 50kg/t, 60kg/t, 70kg/t, 80kg/t, 90kg/t, 100kg/t, 110kg/t, 120 kg/t par rapport au poids total de la phase liquide du milieu réactionnel final. Dans un cas préféré, la concentration en acide téréphtalique obtenue à partir du polyester d'intérêt à l'issue de l'étape de dépolymérisation est comprise entre 100kg/t et 115 kg/t, +/- 10%.

Dans un cas particulier, la concentration en acide téréphtalique total (soluble et non soluble) obtenue à partir du polyester d'intérêt à l'issue de l'étape de dépolymérisation est supérieure à 50kg/t, 60kg/t, 70kg/t, 80kg/t, 90 kg/t, 100kg/t, 110 kg/t, 120 kg/t, 130 kg/t, 140 kg/t, 150 kg/t par rapport au poids total du milieu réactionnel final (phase liquide et phase solide).

Dans un autre cas particulier, la concentration en acide téréphtalique total (soluble et non soluble) obtenue à partir du polyester d'intérêt à l'issue de l'étape de dépolymérisation est supérieure à 50kg/t, 60kg/t, 70kg/t, 80kg/t, 90 kg/t, 100kg/t, 110 kg/t, 120 kg/t, 130 kg/t, 140 kg/t, 150 kg/t par rapport au poids total phase liquide du milieu réactionnel final + AT non soluble.

Avantageusement, selon l'invention, au moins 80% en poids des sels d'AT produits lors de l'étape de dépolymérisation sont sous forme solubilisée, préférentiellement au moins 85%, 90%, 95%.

Dans un cas particulier, la quantité de polyester d'intérêt engagée dans le réacteur est supérieure ou égale à 15% en poids par rapport au poids total du milieu réactionnel initial, le pH est régulé entre 7 et 8 et la température à 60°C +/- 5°C pendant l'étape de dépolymérisation. La concentration en AT dans la phase liquide du milieu réactionnel final après 24h est avantageusement supérieure à 54kg/t. Dans un autre cas particulier, la quantité de polyester d'intérêt engagée dans le réacteur est supérieure ou égale à 15% en poids par rapport au poids total du milieu réactionnel initial, le pH est régulé entre 7,5 et 8,5 et la température à 60°C +/-5°C pendant l'étape de dépolymérisation, et la concentration en AT dans la phase liquide du milieu réactionnel final après 24h est avantageusement supérieure à 77kg/t, et après 48h avantageusement supérieure à 84kg/t.

Dans un autre cas particulier, la quantité de polyester d'intérêt engagée dans le réacteur est supérieure ou égale à 20% en poids par rapport au poids total du milieu réactionnel initial, le pH est régulé entre 7 et 8 et la température à 60°C +/- 5°C pendant l'étape de dépolymérisation. La concentration en AT dans la phase liquide du milieu réactionnel final est avantageusement supérieure à 90kg/t après 24h. Dans un autre cas particulier, la quantité de polyester d'intérêt engagée dans le réacteur est supérieure ou égale à 20% en poids par rapport au poids total du milieu réactionnel initial, le pH est régulé entre 7,5 et 8,5 et la température à 60°C +/- 5°C pendant l'étape de dépolymérisation. La concentration en AT dans la phase liquide du milieu réactionnel final est avantageusement supérieure à 95kg/t après 24h et avantageusement supérieure à 100kg/t après 48h.

Dans un autre cas particulier, la quantité de polyester d'intérêt engagée dans le réacteur est supérieure ou égale à 20% en poids par rapport au poids total du milieu réactionnel initial, le pH est régulé entre 7 et 8, et la température entre 50°C et 60°C pendant l'étape de dépolymérisation. La concentration en AT dans la phase liquide du milieu réactionnel final correspondante après 48h est avantageusement supérieure à 90kg/t. Dans un cas préféré, la température est régulée à 60°C et la concentration en AT dans la phase liquide du milieu réactionnel final après 48h est supérieure à 100kg/t.

Dans un autre cas particulier, la quantité de polyester d'intérêt engagée dans le réacteur est supérieure ou égale à 20% en poids par rapport au poids total du milieu réactionnel initial, le pH est régulé entre 7 et 8, et la température à 60°C pendant l'étape de dépolymérisation, le réacteur utilisé a un volume supérieur à 150L et la concentration en AT dans la phase liquide du milieu réactionnel final après 48h est supérieure à 75kg/t.

Dans un autre cas particulier, la quantité de polyester d'intérêt engagée dans le réacteur est supérieure ou égale à 25% en poids par rapport au poids total du milieu réactionnel initial, le pH est régulé entre 7 et 8, et la température à 60°C pendant l'étape de dépolymérisation, le réacteur utilisé a un volume supérieur à 1000L et la concentration en AT dans la phase liquide du milieu réactionnel final après 48h est supérieure à 120kg/t.

Dans un autre cas particulier, le procédé de production d'acide téréphtalique (AT) selon l'invention à partir d'au moins un PET comprend une étape de dépolymérisation du PET d'une durée inférieure à 48h selon laquelle un matériau plastique contenant du PET est mis en contact avec au moins une cutinase apte à dépolymériser ledit PET dans un réacteur sous agitation, et une étape de récupération de sels d'AT sous forme solubilisée, selon lequel le réacteur contient au début de l'étape de dépolymérisation une quantité de PET engagée supérieure ou égale à 20% en poids par rapport au poids total du milieu réactionnel initial, le pH est régulé entre 7 et 8,5 et la température entre 60°C et 80°C pendant l'étape de dépolymérisation, et la concentration en AT dans le réacteur à la fin de l'étape de dépolymérisation est supérieure à 100kg/t dans la phase liquide du milieu réactionnel final.

Dans un autre cas particulier, le procédé de production d'acide téréphtalique (AT) selon l'invention à partir d'au moins un matériau plastique contenant du PET comprend une étape de dépolymérisation du PET d'une durée inférieure à 48h selon laquelle un matériau plastique contenant du PET est mis en contact avec au moins une cutinase apte à dépolymériser ledit PET dans un réacteur sous agitation, et une étape de récupération de sels d'AT sous forme solubilisée, selon lequel le réacteur contient au début de l'étape de dépolymérisation une quantité de PET engagée comprise entre 15% et 25% en poids par rapport au poids total du milieu réactionnel initial, le pH est régulé entre 7,5 et 8,5 et la température entre 60°C et 80°C pendant l'étape de dépolymérisation, et la concentration en AT dans la phase liquide du milieu réactionnel final dans le réacteur à la fin de l'étape de dépolymérisation est supérieure à 100kg/t.

Dans un autre cas particulier, la quantité de polyester d'intérêt engagée dans le réacteur est comprise entre 15% et 25% en poids par rapport au poids total du milieu réactionnel initial, la solution basique utilisée est concentrée à 15% +/- 1%, et la concentration en AT dans la phase liquide du milieu réactionnel final après 48h est supérieure à 80kg/t, préférentiellement supérieure à 100kg/t. Préférentiellement la quantité de polyester d'intérêt engagée dans le réacteur est supérieure à 20% +/- 2% en poids par rapport au poids total du milieu réactionnel initial, et la concentration en AT dans la phase liquide du milieu réactionnel final après 48h est supérieure à 110kg/t. Avantageusement le pH est régulé entre 7,5 et 8,5.

Dans un autre cas particulier, la quantité de polyester d'intérêt engagée dans le réacteur est comprise entre 15% et 25% en poids par rapport au poids total du milieu réactionnel initial, la solution basique utilisée est concentrée à 20% +/- 1%, et la concentration en AT dans la phase liquide du milieu réactionnel final après 48h est supérieure à 85kg/t, préférentiellement supérieure à 110kg/t. Préférentiellement la quantité de polyester d'intérêt engagée dans le réacteur est supérieure à 20% +/- 2%, et la concentration en AT dans la phase liquide du milieu réactionnel final après 48h est supérieure à 110kg/t. Avantageusement le pH est régulé entre 7,5 et 8,5.

Dans un autre cas particulier, la quantité de polyester d'intérêt engagée dans le réacteur est comprise entre 15% et 25% en poids par rapport au poids total du milieu réactionnel initial, la solution basique utilisée est concentrée à 25% +/- 1%, et la concentration en AT dans la phase liquide du milieu réactionnel final après 48h est supérieure à 90kg/t, préférentiellement supérieure à 110kg/t. Préférentiellement la quantité de polyester d'intérêt engagée dans le réacteur est supérieure à 20% +/- 2%, et la concentration en AT dans la phase liquide du milieu réactionnel final après 48h est supérieure à 110kg/t. Avantageusement le pH est régulé entre 7,5 et 8,5.Dans un autre cas particulier, le matériau plastique est sélectionné parmi des fibres et/ou des déchets fibres et/ou textiles, et la quantité de polyester d'intérêt engagée dans le réacteur est comprise entre 15% et 25% en poids par rapport au poids total du milieu réactionnel initial, la solution basique utilisée est concentrée à 20% +/- 1%, et la concentration en AT dans la phase liquide du milieu réactionnel final après 48h est supérieure à 80kg/t, préférentiellement supérieure à 90kg/t. Avantageusement le pH est régulé entre 7,5 et 8,5.

Selon l'invention, il est possible de récupérer aisément les sels de téréphtalate (et/ou l'acide téréphtalique) solubilisé(s) dans la phase liquide du milieu réactionnel final à partir du contenu du réacteur. Dans un mode particulier, l'étape de récupération des sels d'AT solubilisés comprend notamment une séparation de la phase liquide contenant les sels d'AT du reste du milieu réactionnel. Dans un cas particulier, cette étape de séparation des sels de téréphtalate solubilisé(s) dans la phase liquide est réalisée par filtration du milieu réactionnel permettant la récupération, dans une solution, des sels de téréphtalate sous forme solubilisée. Le seuil de coupure de la filtration peut être adapté par l'homme de l'art. L'étape de séparation peut également être réalisée par centrifugation ou toute autre technique connue de l'homme de l'art.

Le résidu de séparation (« retentât » i.e. la phase solide comprenant notamment le polyester résiduel non dégradé et/ou les autres polymères contenus dans le matériau plastique et/ou des sels d'AT et AT non solubilisé(s)) peut être recyclé dans le réacteur afin de subir une nouvelle étape de dépolymérisation. Il peut également être lavé avec de l'eau afin de permettre la dissolution des sels d'AT non solubilisé(s) dans la phase liquide et ainsi permettre leur récupération sous forme solubilisée dans l'eau de lavage.

Avantageusement, les sels d'AT récupérés peuvent être réutilisés sous forme d'AT, notamment pour la production de nouveaux polyesters. Selon l'invention, le procédé comprend une étape supplémentaire de récupération de l'AT par précipitation de l'AT contenu dans lesdits sels.

Dans un mode particulier, cette précipitation de l'AT est réalisée par acidification du milieu. Par exemple, la solution filtrée (i.e. la phase liquide du milieu réactionnel final) contenant les sels de téréphtalate solubilisé(s) peut être soumise à tout ou partie des étapes suivantes (cette suite d'étapes étant également adaptée à l'eau de lavage citée ci-dessus) :
1. Purification de la solution filtrée (et/ou eau de lavage) par soumission de la solution à une ou plusieurs étapes sélectionnées parmi l'ultrafiltration, la décoloration sur charbon, le passage sur échangeurs d'ions et la chromatographie ; et/ou
2. Précipitation de l'acide téréphtalique contenu dans la solution filtrée, l'eau de lavage ou la solution purifiée par acidification de la solution avec un acide minéral (pouvant par exemple être sélectionné parmi les acides suivants : acide sulfurique, acide chlorhydrique, acide phosphorique, ou acide nitrique) ou avec un acide organique (de type acide acétique), seul ou en mélange. La solution peut également être acidifiée par surpression de CO₂. Cette étape induit également une solubilisation des sels produits en même temps que la précipitation de l'AT ; et/ou
3. Filtration de la solution contenant l'acide téréphtalique précipité pour récupérer de l'acide téréphtalique sous forme solide ; et/ou
4. Lavage (préférentiellement plusieurs lavages successifs) de l'acide téréphtalique avec de l'eau purifiée et séchage pour obtenir de l'AT purifié (« CTA »).

Le CTA obtenu peut ensuite être cristallisé et éventuellement purifié de manière additionnelle pour obtenir de l'AT purifié et cristallisé (« PTA ») par toutes techniques connues de l'homme du métier.

Le CTA et/ou le PTA issus du procédé de l'invention peuvent être réutilisés seuls ou en mélange. Ils peuvent notamment être repolymérisés, seuls ou en mélange, pour la synthèse d'un polyester contenant au moins une unité d'acide téréphtalique, identique ou différent du polyester d'intérêt engagé dans le réacteur.

Les sels et le(s) autre(s) monomère(s) obtenu(s) dans le filtrat issu de l'étape 3 peuvent être extraits et purifiés par des techniques connues de l'homme de l'art afin d'être réutilisés et/ou valorisés.

Dans un autre mode de mise en œuvre, la solution filtrée (i.e. la phase liquide du milieu réactionnel final) contenant les sels de téréphtalate solubilisés est soumise à une étape de concentration pouvant être réalisée par toutes méthodes permettant d'éliminer l'eau contenue dans la solution (ex : évaporation) et entraînant ainsi la précipitation des sels de téréphtalate sous forme solide. Les sels d'AT sous forme solide sont récupérés par filtration puis remis en solution avant acidification de la solution par un acide (minéral ou organique) pour précipiter de l'acide téréphtalique. Cette étape de concentration peut être réalisée à tout moment dans le déroulé du procédé de purification et sera suivie d'une étape d'acidification du milieu. L'étape 4 citée ci-dessus peut ensuite être réalisée de manière à obtenir le CTA.

Selon l'invention, il est possible de récupérer au moins un autre monomère issu de la dépolymérisation du polyester d'intérêt. Le procédé selon l'invention comprend ainsi une étape supplémentaire selon laquelle on récupère au moins un autre monomère constituant le polyester d'intérêt. Dans un mode de réalisation, le polyester d'intérêt est le PET, et on récupère à l'issue de l'étape de dépolymérisation des monomères d'éthylène glycol en plus de l'acide téréphtalique. Dans un autre mode de réalisation, le polyester d'intérêt est le PTT, et on récupère à l'issue de l'étape de dépolymérisation des monomères de propanediol (ou propylène glycol) en plus de l'acide téréphtalique. Dans un autre mode de réalisation, le polyester d'intérêt est le PBT, et on récupère à l'issue de l'étape de dépolymérisation des monomères de butanediol en plus de l'acide téréphtalique. Dans un autre mode de réalisation, le polyester d'intérêt est le PBAT, et on récupère à l'issue de l'étape de dépolymérisation des monomères de butanediol et/ou d'acide adipique en plus de l'acide téréphtalique. Dans un autre mode de réalisation, le polyester d'intérêt est le PCT, et on récupère à l'issue de l'étape de dépolymérisation des monomères de cyclohexanediméthanol en plus de l'acide téréphtalique. Dans un autre mode de réalisation, le polyester d'intérêt est le PEIT, et on récupère à l'issue de l'étape de dépolymérisation des monomères d'éthylène glycol et/ou d'isosorbide en plus de l'acide téréphtalique.

Selon l'invention, en plus de l'acide téréphtalique, il est également possible de récupérer des oligomères, i.e. des molécules comprenant entre 2 et 20 monomères, dont au moins une unité d'acide téréphtalique. Dans un cas particulier, le polyester d'intérêt est le PET et on récupère à l'issue de l'étape de dépolymérisation des oligomères tels que le methyl-2-hydroxyethyl téréphtalate (MHET), bis(2-hydroxyethyl) téréphtalate (BHET), et diméthyle téréphtalate (DMT) en plus de l'acide téréphtalique.

L'invention a pour objet l'utilisation d'un réacteur présentant un volume supérieur à 1000L, 10 000 L, 100 000 L, 400 000 L pour la mise en œuvre d'un procédé de production d'acide téréphtalique (AT) décrit ci-dessus.

L'invention a également pour objet un réacteur d'un volume d'au moins 1000 Litres contenant au moins un polyester d'intérêt comprenant au moins une unité d'AT et au moins une enzyme apte à dépolymériser ledit polyester d'intérêt, et dans lequel se déroule au moins une étape de dépolymérisation enzymatique dudit polyester d'intérêt, la quantité de polyester engagée dans le réacteur étant supérieure ou égale à 11% en poids par rapport au poids total du milieu réactionnel initial, et la concentration en AT dans la phase liquide du milieu réactionnel final étant supérieure à 40kg/t. Avantageusement, la quantité de polyester engagée dans le réacteur est comprise entre 15% et 25% en poids par rapport au poids total du milieu réactionnel initial, la concentration en AT dans la phase liquide du milieu réactionnel final est supérieure à 100kg/t, et le pH est régulé pendant l'étape de dépolymérisation entre 7,5 et 8,5 par l'ajout d'une solution basique concentrée entre 15% et 50% en poids de base par rapport au poids total de la solution basique, préférentiellement par l'ajout d'une solution basique concentrée entre 15% et 25%.

Selon l'invention, le procédé est mis en œuvre de manière discontinue, sous forme de « batch » (traitement par lot). D'une manière générale, le procédé comprend donc une étape de dépolymérisation conduite pendant un temps déterminé à partir d'un volume de milieu réactionnel initial déterminé, suivie d'une étape de récupération des sels d'AT produits. A l'issue de l'étape de dépolymérisation, le réacteur peut être vidangé de manière à récupérer l'ensemble du milieu réactionnel, ce dernier pouvant ensuite subir les différentes étapes décrites ci-dessus de manière à séparer les sels de téréphtalate solubilisé(s) du reste du milieu réactionnel, les purifier et récupérer l'AT.

L'ensemble des caractéristiques du procédé de production d'acide téréphtalique selon l'invention décrit ci-dessus peut également être appliqué à un procédé de production de monomères proches de l'acide téréphtalique, et particulièrement à un procédé de production d'acide 2,5-furandicarboxylique (FDCA) à partir de polyéthylène furanoate (PEF).

### EXEMPLES

### Exemple 1 : Production d'acide téréphtalique dans un réacteur comprenant une quantité de PET engagée supérieure à 10% en poids par rapport au poids total du milieu réactionnel initial

Pour ce plan d'expérience, la production d'acide téréphtalique a été réalisée dans des réacteurs agités à fond plat, d'un volume total de 500 mL (MiniBioréacteurs, Global Process Concept).

Chaque réacteur était équipé d'une sonde température et d'une sonde pH (Hamilton, EAsyFerm HB BioArc 120). La régulation de ces deux paramètres aux valeurs de consignes était assurée par des contrôleurs PID internes au logiciel C-bio (Global Process Concept). Une pâle marine de diamètre 3 cm fixée à l'arbre central tournant à 300, 400 ou 450 rpm a permis l'agitation du milieu réactionnel. Plusieurs solutions basiques ont été utilisées pour la régulation du pH : soit du NaOH 6M (c'est à dire concentrée à 19,4%), soit du NH₄OH 6M (concentrée à 17,4%), soit du KOH 6M (concentrée à 25%).

Pour les expériences A à C, la production d'acide téréphtalique (AT) a été réalisée à partir de préformes de bouteilles incolores, composées à 100% de PET amorphe, réduites à l'état de poudre fine par cryobroyage (D50 = 850µm).

Pour les expériences D à K, la production d'acide téréphtalique a été réalisée à partir de paillettes plastiques colorées et lavées issues de la filière de recyclage de déchets plastiques en PET, qui nous ont été gracieusement données. Ces matériaux plastiques, composés à 98% m/m de PET, ont subi une étape d'extrusion, suivie d'un refroidissement rapide permettant l'amorphisation du PET contenu dans les déchets. L'extrudeuse utilisée pour l'amorphisation était une extrudeuse double vis ZSE 18 MAXX de chez Leistritz. La température des zones de chauffe a été réglée à 260°C sur les 4 premières zones et à 250°C sur les 6 dernières et une vitesse de rotation de vis de 200 rpm. Le jonc arrivant en tête d'extrudeuse est ensuite immédiatement immergé dans un bain d'eau à 10°C. Le degré de cristallinité du PET à l'issue de cette étape d'amorphisation a été évalué à environ 19% (par DSC). Le jonc obtenu a été granulé puis réduit à l'état de poudre fine à l'aide d'un microniseur (grille 1mm). La poudre a ensuite été soumise à un tamis de 500µm pour ne récupérer que les poudres inférieures à cette taille.

L'enzyme utilisée était la LC-Cutinase, enzyme connue comme apte à dépolymériser le PET (SEQ ID N°1, correspondant aux acides aminés 36 à 293 de la séquence décrite dans Sulaiman et al., Appl Environ Microbiol. 2012 Mar). Elle a été produite par fermentation d'un microorganisme recombinant en milieu liquide. L'enzyme dégradant le PET est ajoutée selon un ratio en poids de 1/1000 ou 2/1000 par quantité de PET engagée.

Ces produits plastiques ou déchets plastiques ont été introduits dans le réacteur de sorte que la quantité de PET engagé au début de l'étape de dépolymérisation soit comprise entre 5% et 40% par rapport au poids total du milieu réactionnel initial. Du tampon phosphate est ajouté aux matériaux plastiques et à l'enzyme afin d'atteindre le poids total du milieu réactionnel initial.

L'ensemble des paramètres associés à l'étape de dépolymérisation des différents procédés testés est repris dans les Tableaux 1A et 1B ci-dessous :

**Tableau 1A : Paramètres utilisés durant les procédés A-C de production d'AT à partir de préformes bouteille**

| Tests | A | B | C |
|---|---|---|---|
| Matériaux plastiques utilisés comprenant le polyester d'intérêt | Préforme Bouteille - 100% PET - Micronisées (< 1mm) | | |
| PET engagé sur poids total du milieu réactionnel initial | 5,0% | 15,0% | 20,0% |
| Poids total du milieu réactionnel initial (g) | 237 | 265 | 281 |
| Ratio en poids Enzyme/PET | 1/1000 | | |
| Liquide du milieu réactionnel | Tampon phosphate 10 mM pH 7 | | |
| Température durant l'étape de dépolymérisation | 60°C | | |
| Agitation (rpm) durant l'étape de dépolymérisation | 300 | | |
| Consigne régulation pH durant l'étape de dépolymérisation | 7,00 | | |
| Solution basique utilisée pour la régulation pH | NH₄OH 6M (17,4%) | | |

**Tableau 1B : Paramètres utilisés durant les procédés D-K de production d'AT à partir de déchets plastiques**

| Tests | D | E | F | G | H | I | J | K |
|---|---|---|---|---|---|---|---|---|
| Matériaux plastiques utilisés comprenant le polyester d'intérêt | Paillettes colorées lavées - 98% PET Amorphisé -Micronisées (<500µm) | | | | | | | |
| PET engagé sur poids total du milieu réactionnel initial | 10,0% | 20,0% | 25,0% | 30,0% | 40,0% | 20,0% | 30,0% | 40,0% |
| Poids total du milieu réactionnel initial (g) | 282 | 281 | 281 | 282 | 240 | 281 | 281 | 280 |
| Ratio en poids Enzyme/PET | 2/1000 | | | | | | | |
| Liquide du milieu réactionnel | Tampon Phosphate 100mM pH8 | | | | | | | |
| Température durant l'étape de dépolymérisation | 60°C | | | | | | | |
| Agitation (rpm) durant l'étape de dépolymérisation | 300 | | | | 400 | 300 | 450 | 450 |
| Consigne régulation pH durant l'étape de dépolymérisation | 8,00 | | | | | | | |
| Solution basique utilisée pour la régulation pH | NaOH 19,4% (6M) | | | | | KOH 25% (6M) | | |

Des prélèvements réguliers ont permis de suivre la cinétique de production d'acide téréphtalique. La phase solide (comprenant les matériaux plastiques non dégradés) a d'abord été séparée de la phase liquide, contenant des sels de téréphtalate sous forme soluble, par centrifugation (D100- DragonLab).

La concentration en AT a été déterminée par chromatographie (UHPLC). Pour cela, 1 mL de méthanol et 100 µL de HCl 6M ont été ajoutés à l'échantillon dilué afin de décomplexer les sels d'AT. Si nécessaire, des dilutions ont été réalisées sur les prélèvements avec de l'eau osmosée. L'échantillon ainsi préparé a été filtré sur filtre cellulosique 0.22µM et 20 µL ont été injectés sur la colonne chromatographique. Le système HPLC utilisé était le modèle 3000 UHPLC system (Thermo Fisher Scientific, Inc. Waltham, MA, USA), comprenant une pompe, un système d'échantillonnage automatique, une colonne thermostaté à 25°C et un détecteur UV à 240nm. Trois éluant ont été utilisés : 10 mM H2SO4 (éluant A) ; de l'eau ultrapure (éluant B) et du méthanol (éluant C). L'acide téréphtalique est séparé des autres molécules par un gradient entre ces trois solvants. L'acide téréphtalique est mesuré selon les étalons standards préparés à partir d'acide téréphtalique commercial (Acros Organics).

La concentration d'acide téréphtalique produit au bout de 24h pour les différents tests est indiquée dans les Tableaux 2A et 2B ci-dessous.

**Tableau 2A : Concentration et fraction soluble d'acide téréphtalique obtenu à l'issue des étapes de dépolymérisation dont les paramètres sont décrits au Tableau 1A.**

| Tests | A | B | C |
|---|---|---|---|
| Matériaux plastiques utilisés comprenant le polyester d'intérêt | Préformes Bouteille - 100% PET - Micronisées (< 1mm) | | |
| PET engagé sur poids total du milieu réactionnel initial | 5,0% | 15,0% | 20,0% |
| AT kg/t par rapport au poids total de la phase liquide du milieu réactionnel final - 24h | 14,4 | 54,2 | 68,7 |
| Fraction de sels d'AT Soluble | 100% | 100% | 100% |

Le procédé de production d'acide téréphtalique selon l'invention permet donc d'atteindre au bout de 24h une concentration en AT supérieure à 54kg/t et 69kg/t, dans des réacteurs contenant au début de l'étape de dépolymérisation une quantité de polyester engagée respectivement égale à 15% et 20% en poids par rapport au poids total du milieu réactionnel initial, le pH étant régulé à 7 pendant l'étape de dépolymérisation.

**Tableau 2B : Concentration et fraction soluble d'acide téréphtalique obtenues à l'issue des étapes de dépolymérisation dont les paramètres sont décrits au Tableau 1B**

| Tests | D | E | F | G | H | I | J | K |
|---|---|---|---|---|---|---|---|---|
| Matériaux plastiques utilisés comprenant le polyester d'intérêt | Paillettes colorées lavées - 98% PET Amorphisé -Micronisées (<500µm) | | | | | | | |
| PET engagé sur poids total du milieu réactionnel initial | 10,0% | 20,0% | 25,0% | 30,0% | 40,0% | 20,0% | 30,0% | 40,0% |
| AT total (soluble et non soluble) kg/t de [phase liquide du milieu réactionnel final + AT non soluble]- 24h | 56,9 | 95,8 | 106,1 | 109,8 | 131,5 | 77,2 | 94,9 | 118,5 |
| AT kg/t de phase liquide du milieu réactionnel final - 24h | 56,9 | 95,8 | 106,1 | 109,8 | 119,7 | 77,2 | 94,9 | 118,5 |
| Fraction de sels d'AT Soluble | 100% | 100% | 100% | 100% | 91% | 100% | 100% | 100% |

Le procédé de production d'acide téréphtalique selon l'invention permet donc d'atteindre au bout de 24h une concentration en AT supérieure à 57kg/t, 77kg/t, 106kg/t, 95kg/t et 118kg/t dans des réacteurs contenant au début de l'étape de dépolymérisation une quantité de polyester engagée respectivement égale à 10%, 20%, 25%, 30% et 40% en poids par rapport au poids total du milieu réactionnel initial, le pH étant régulé à 8 pendant l'étape de dépolymérisation.

### Exemple 2 : Production d'acide téréphtalique dans un réacteur comprenant une quantité de PET engagée égale à 20% en poids par rapport au poids total du milieu réactionnel initial, le pH et la température étant régulés, pendant l'étape de dépolymérisation, à des valeurs fixées respectivement entre 7 et 8, et entre 40°C et 60°C.

Pour ce second plan d'expérience, l'ensemble des tests s'est déroulé dans les mêmes réacteurs agités que ceux décrits dans l'Exemple 1 avec une agitation durant l'étape de dépolymérisation à 300 rpm. La solution basique utilisée pour la régulation du pH était du NaOH 19,4% (6M).

La production d'acide téréphtalique (AT) a été réalisée à partir de paillettes plastiques colorées et lavées issues de la filière de recyclage de déchets plastiques en PET identiques à celles utilisées pour les expériences D à H de l'Exemple 1, à l'exception du tamisage final. Les poudres obtenues ont donc une granulométrie inférieure à 1mm. La part PET de ces déchets plastiques constituait 20% de la masse engagée au début de l'étape de dépolymérisation du PET par rapport au poids total du milieu réactionnel initial.

L'enzyme utilisée est identique à celle utilisée dans l'Exemple 1. Elle a été ajoutée selon un ratio en poids de 2/1000 par quantité de PET engagé.

Du tampon phosphate 100 mM pH8 est ajouté aux matériaux plastiques et à l'enzyme afin d'atteindre le poids total du milieu réactionnel initial.

Trois températures de consigne ont été testées, ainsi que trois valeurs de pH. Les informations principales sont résumées dans le Tableau 3 suivant :

**Tableau 3 : Paramètres utilisés durant les différents procédés de production d'AT à partir de déchets plastiques introduits à hauteur de 20% de PET engagé sur poids total du milieu réactionnel initial.**

| Tests | A | B | C | D | E |
|---|---|---|---|---|---|
| Matériaux plastiques utilisés comprenant le polyester d'intérêt | Paillettes plastiques colorées lavées - 98% PET Amorphisé - Micronisées (< 1mm) | | | | |
| PET engagé sur poids total du milieu réactionnel initial | 20,0% sur 281 g | | | | |
| Température (°C) durant l'étape de dépolymérisation | 60 °C | 50 °C | 40 °C | 60 °C | |
| Consigne régulation pH durant l'étape de dépolymérisation | 8.0 | | | 7.0 | 7.5 |

Des prélèvements réguliers ont permis de suivre la cinétique de production d'acide téréphtalique et la concentration en AT a été déterminée de manière similaire à celle de l'Exemple 1.

Pour les conditions décrites précédemment, les résultats suivants ont été obtenus à 48h ou à 72h.

**Tableau 4 : Concentration d'acide téréphtalique obtenu à l'issue des étapes de dépolymérisation au début de laquelle la quantité de PET engagé est égale à 20% en poids par rapport au poids du milieu réactionnel initial, et dont les paramètres température et pH ont été régulés tels que décrits dans le Tableau 3.**

| Tests | A | B | C | D | E |
|---|---|---|---|---|---|
| Température (°C) durant l'étape de dépolymérisation | 60 °C | 50 °C | 40 °C | 60 °C | |
| Consigne régulation pH durant l'étape de dépolymérisation | 8.0 | | | 7.0 | 7.5 |
| AT kg/t par rapport au poids total de la phase liquide du milieu réactionnel final à 48h | 114 | / | / | 104 | 106 |
| AT kg/t par rapport au poids total de la phase liquide du milieu réactionnel à 72h | 113 | 94 | 41 | / | / |

Le procédé de production d'acide téréphtalique selon l'invention réalisé dans un réacteur contenant au début de l'étape de dépolymérisation une quantité de polyester engagée respectivement égale à 20%, permet d'atteindre au bout de 48h et 72h une concentration en AT supérieure à 40kg/t. A 60°C, ce procédé permet d'atteindre au bout de 48h une concentration supérieure à 90kg/t pour un pH entre 7 et 8. A 50°C, ce procédé permet d'atteindre au bout de 72h une concentration supérieure à 90kg/t pour un pH entre 7 et 8.

### Exemple 3 : Production d'acide téréphtalique dans un réacteur comprenant une quantité de PET engagée égale à 20% en poids, contenu dans un matériau plastique sous forme de granules.

Pour ce troisième plan d'expérience, l'ensemble des tests s'est déroulé dans les mêmes réacteurs agités que ceux décrits dans l'Exemple 1. La consigne de température a été fixée à 60°C et le pH a été régulé à 8.0 en utilisant du NaOH 19,4% (6M) comme solution basique.

La production d'acide téréphtalique (AT) a été réalisée à partir de PET Lighter C93 de chez Resinex. Le PET a été amorphisé par extrusion, suivi d'un refroidissement rapide. L'extrudeuse utilisée pour l'amorphisation était une extrudeuse double vis ZSE 18 MAXX de chez Leistritz, dont les zones de chauffe ont été réglées entre 285°C et 304°C. Le degré de cristallinité du PET obtenu sous forme de granules à l'issue de cette étape d'amorphisation a été évalué à environ 13% (par DSC).

Les granules ont été introduits dans le réacteur à hauteur de 20% en poids par rapport au poids du milieu réactionnel initial. L'enzyme utilisée est identique à celle utilisée dans l'Exemple 1. Elle a été ajoutée selon un ratio en poids de 1/1000 de PET engagé. Du tampon phosphate de potassium à 10 mM pH8 est ajouté aux matériaux plastiques et à l'enzyme afin d'atteindre le poids total du milieu réactionnel initial.

Des prélèvements réguliers tels que décrits dans l'Example 1 ont permis de suivre la cinétique de production d'acide téréphtalique. L'acide téréphtalique produit a été mesuré par HPLC selon le protocole décrit dans l'Exemple 1. Au bout de 88h, on obtient 62 kg/t d'AT par rapport au poids total de la phase liquide du milieu réactionnel final. Les résultats indiquent qu'il est également possible d'atteindre les performances revendiquées dans cette demande lorsque le matériau plastique contenant le polyester d'intérêt est introduit sous forme de granules.

### Exemple 4 : Production d'acide téréphtalique à différentes échelles de réacteurs.

Dans cet exemple, différents réacteurs agités ont été utilisés pour réaliser la production d'acide téréphtalique. Ces réacteurs de tailles croissantes permettent de valider la montée en échelle du procédé et son utilisation à une échelle industrielle ou semi-industrielle.

Pour ces tests, deux types de matériaux plastiques ont été utilisés. Dans les tests A, B, C, E et F il s'agit des paillettes colorées lavées décrites dans l'Exemple 1 (98% de PET Amorphisé, micronisées <500µm), alors que pour l'exemple D, il s'agit de préformes de bouteilles également décrites dans l'Exemple 1 (100% PET, micronisées < 1mm). Ces matériaux plastiques sont engagés de manière à obtenir une quantité de PET engagée de 20% en poids par rapport au poids du milieu réactionnel initial. La consigne de température a été fixée à 60°C et le pH a été régulé à 8.0 ou à 7.0 à l'aide de NaOH 19,4% (Tests A à D) ou NaOH 25% m/m (Test E et F). Pour les tests A, B, C, D et E, l'enzyme utilisée est identique à celle utilisée dans l'Exemple 1. Pour le test F, il s'agit d'un variant de l'enzyme de l'exemple 1 (avec les mutations suivantes SEQ ID N°1 + F208I + D203C + S248C + V170I + Y92G) également obtenue par fermentation d'un microorganisme recombinant. Elles ont été ajoutées à hauteur d'un ratio en poids de 1/1000 par poids de PET engagé pour les tests A à D et de 2/1000 pour les tests E et F.

Pour le test A, un réacteur à fond plat de 500 mL décrit dans l'Exemple 1 a été utilisé. L'agitation est assurée par une pâle marine de diamètre 3 cm, fixée sur l'arbre central. La vitesse d'agitation a été fixée à 300 rpm. 56.3 g de matériaux plastiques ont été engagés.

Pour le test B, un réacteur à fond bombé d'un volume total de 5L (Global Process Concept) a été utilisé. Le réacteur était équipé d'une sonde température et d'une sonde pH (Hamilton, EasyFerm HB BioArc 325). La régulation de ces deux paramètres aux valeurs de consignes était assurée par des contrôleurs PID interne au logiciel C-bio (Global Process Concept). Une pâle marine de diamètre 5.5 cm fixé à l'arbre central tournant à 200 rpm a permis l'agitation du milieu réactionnel. 375 g de matériaux plastiques ont été engagés.

Pour le test C, un réacteur à fond bombé d'un volume total de 40L a été utilisé. Le réacteur était équipé d'une sonde température et d'une sonde pH (Rosemount analytical HX338-05). Une pâle marine de diamètre 14 cm fixé à l'arbre central tournant à 150 rpm a permis l'agitation du milieu réactionnel. 4kg de matériaux plastiques ont été engagés.

Pour le test D, un réacteur à fond bombé d'un volume total de 150L a été utilisé. Le réacteur était équipé d'une sonde température et d'une sonde pH (Easy Ferm bioArc 120, Hamilton). Une pâle marine de diamètre 25 cm fixé à l'arbre central tournant à 80 rpm a permis l'agitation du milieu réactionnel. 14kg de matériaux plastiques ont été engagés.

Pour les tests E et F, un réacteur à fond plat d'un volume total de 1000 L a été utilisé. Le réacteur était équipé d'une sonde température et d'une sonde pH (In Pro3100/SG/325, Mettler Toledo). Une pâle marine de diamètre variable a permis l'agitation du milieu réactionnel. 75kg de matériaux plastiques ont été engagés.

Il est entendu que la forme bombée ou plate du fond du réacteur n'a aucune incidence sur le procédé et que la forme des fonds est interchangeable.

Les principales informations ont été résumées dans le Tableau 5 suivant :

**Tableau 5 : Paramètres utilisés durant les différents procédés de production d'AT à partir de matériaux plastiques introduits à hauteur de 20% de PET engagé sur poids total du milieu réactionnel initial, dans des réacteurs de 500mL à 1000L.**

| Tests | A | B | C | D | E | F |
|---|---|---|---|---|---|---|
| Taille du réacteur | 500mL | 5L | 40L | 150L | 1000L | |
| Matériaux plastiques utilisés comprenant le polyester d'intérêt | Paillettes colorées lavées - 98% PET Amorphisé - Micronisées (<500 µm) | | | Préformes Bouteille - 100% PET - Micronisées (<1mm) | Paillettes colorées lavées | |
| PET engagé sur poids total du milieu réactionnel initial | 20% | | | | | |
| Liquide du milieu réactionnel | Tampon phosphate 10mM | | Eau osmosée | Eau osmosée | Eau osmosée | Eau réseau |
| Agitation (rpm) durant l'étape de dépolymérisation | 300 | 200 | 150 | 80 | 100rpm | 100rpm |
| Consigne régulation pH durant l'étape de dépolymérisation | 8.0 | | | 7.0 | 8.0 | 8.0 |

Des prélèvements réguliers tel que décrits dans l'Example 1 ont permis de suivre la cinétique de production d'acide téréphtalique. La concentration en AT a été déterminée par chromatographie UHPLC (décrit dans l'Exemple 1).

Ainsi pour les différentes réactions, les résultats obtenus sont détaillés dans le Tableau 6:

**Tableau 6 : Concentration d'acide téréphtalique obtenu à l'issu des étapes de dépolymérisation des procédés décrits dans le Tableau 5.**

| Tests | A | B | C | D | E | F |
|---|---|---|---|---|---|---|
| Taille du réacteur | 500mL | 5L | 40L | 150L | 1000L | 1000L |
| Temps (h) | 16 | 16 | 30 | 48 | 48 | 44 |
| AT total (soluble et non soluble) en kg/t de [phase liquide du milieu réactionnel final + AT non soluble] | 91 | 90 | 101 | 78 | 122 | 116 |
| AT kg/t par rapport au poids total de la phase liquide du milieu réactionnel final | 91 | 90 | 101 | 78 | 115,9 | 113,7 |
| Fraction de sels d'AT Soluble | 100% | 100% | 100% | 100% | 95% | 98% |

Ainsi des concentrations supérieures à 78kg/t d'acide téréphtalique par rapport au poids total de la phase liquide du milieu réactionnel final sont obtenues dans chacune des conditions décrites. Particulièrement, des concentrations supérieures à 110kg/t d'acide téréphtalique par rapport au poids total de la phase liquide du milieu réactionnel final sont obtenues dans des réacteurs de 1000L.

### Exemple 5 : production d'acide téréphtalique dans un réacteur contenant du PET issu de déchets textiles

Pour ces expériences, la production d'acide téréphtalique a été réalisée dans des réacteurs à fond bombé d'un volume total de 5L (Global Process Concept) (décrit dans l'Exemple 4).

Pour l'expérience A, la production d'acide téréphtalique a été réalisée à partir de textiles d'habillement usagés, déchiquetés et dépourvus des métaux et des points durs (boutons, fermetures éclairs, etc...). Les morceaux de textile déchiquetés ont une taille de 5 x 5 cm et contiennent environ 83% de PET.

Pour l'expérience B, la production d'AT a été réalisée à partir de rebus de production d'un procédé de tissage à jet d'eau, dont la matière est sous forme d'amas de fils continus et contient environ 100% de PET.

Ces matières textiles ont subi une étape de séchage à 60°C pendant 16h puis une étape d'extrusion, suivie d'un refroidissement rapide permettant l'amorphisation du PET contenu dans les déchets. La même extrudeuse que pour l'exemple 1 été utilisée. Les températures des zones de chauffe ont été réglées selon le profil suivant :
265°C-265°C-265°C-255°C-255°C-250°C-250°C-245°C-245°C-245°C

La vitesse de rotation des vis a été réglée à 150 rpm. L'introduction de la matière dans l'extrudeuse a été effectuée manuellement. L'étape de refroidissement et l'étape de réduction en poudre sont identiques à celles utilisées dans l'exemple 1. Le degré de cristallinité des échantillons a été évalué à environ 18% pour l'échantillon de l'expérience A et est inférieur à 10% pour l'échantillon de l'expérience B.

L'enzyme utilisée est identique à celle utilisée dans l'Exemple 4 pour le test F. Ces produits plastiques ou déchets plastiques ont été introduits dans le réacteur de sorte que la quantité de PET engagée au début de l'étape de dépolymérisation est comprise entre 16,6% et 20% par rapport au poids total du milieu réactionnel initial. Du tampon phosphate est ajouté aux matériaux plastiques et à l'enzyme afin d'atteindre le poids total du milieu réactionnel initial.

L'ensemble des paramètres associés à l'étape de dépolymérisation des expériences A et B, est repris dans le Tableau 7

**Tableau 7 : Paramètres utilisés durant les expériences A et B de production d'AT à partir de déchets textiles.**

| Tests | A | B |
|---|---|---|
| Matériaux plastiques utilisés comprenant le polyester d'intérêt | Vêtements usagés déchiquetés amorphisés -micronisés (<500µm) - 83% PET | Rebus de tissage amorphisés -micronisés (<500µm) - 100% PET |
| PET engagé sur poids total du milieu réactionnel initial | 16.6% | 20.00% |
| Poids total du milieu réactionnel initial (g) | 2600 | 2600 |
| Ratio en poids Enzyme/PET | 2/1000 | 2/1000 |
| Liquide du milieu réactionnel | Tampon Phosphate 100mM pH8 | Tampon Phosphate 100mM pH8 |
| Température durant l'étape de dépolymérisation | 60°C | 60°C |
| Agitation (rpm) durant l'étape de dépolymérisation | 300 | 300 |
| Consigne régulation pH durant l'étape de dépolymérisation | 8.0 | 8.0 |
| Solution basique utilisée pour la régulation pH | NaOH 19,4% | NaOH 19,4% |

Des prélèvements réguliers tels que décrits dans l'Example 1 ont permis de suivre la cinétique de production d'acide téréphtalique. L'acide téréphtalique produit a été mesuré par HPLC selon le protocole décrit dans l'Exemple 1

Les concentrations d'acide téréphtalique produit au bout de 24h et 48h pour les différents tests sont indiquées dans le Tableau 8 ci-dessous.

**Tableau 8: Concentration d'acide téréphtalique obtenue à l'issu des étapes de dépolymérisation dont les paramètres sont décrits au Tableau 7.**

| Tests | A | B |
|---|---|---|
| AT kg/t par rapport au poids total de la phase liquide du milieu réactionnel final - 24h | 77 | 95 |
| AT kg/t par rapport au poids total de la phase liquide du milieu réactionnel final - 48h | 84 | 102 |

Ainsi des concentrations supérieures à 75kg/t d'acide téréphtalique par rapport au poids total de la phase liquide du milieu réactionnel final sont obtenues dans chacune des conditions décrites.

## Revendications

1. Procédé de production d'acide téréphtalique (AT) à partir d'au moins un polyester d'intérêt comprenant au moins une unité d'AT, comprenant une étape de dépolymérisation enzymatique du polyester, selon laquelle ledit polyester est mis en contact avec au moins une enzyme apte à dépolymériser ledit polyester dans un réacteur sous agitation et une étape de récupération de sels d'AT sous forme solubilisée, **caractérisé en ce que** la quantité de polyester engagée dans le réacteur est supérieure ou égale à 11% en poids par rapport au poids total du milieu réactionnel initial, **en ce que** le pH est régulé entre 6,5 et 9 pendant l'étape de dépolymérisation, **en ce que** la température est régulée entre 35°C et 90°C pendant l'étape de dépolymérisation, **en ce que** l'étape de dépolymérisation dure entre 1h et 120h, et **en ce que** la concentration en AT dans la phase liquide du milieu réactionnel final est supérieure à 40kg/t.

2. Procédé selon la revendication 1, **caractérisé en ce que** le ratio en poids quantité d'enzyme engagée sur quantité de polyester d'intérêt engagée est compris entre 0,01/1000 et 3/1000, préférentiellement entre 1/1000 et 2/1000, et/ou **en ce que** le polyester d'intérêt est sous forme de poudre, préférentiellement de granulométrie inférieure à 2 mm, plus préférentiellement inférieure à 1 mm.

3. Procédé selon la revendication 1 ou 2, **caractérisé en ce que** le polyester d'intérêt est choisi parmi PTT, PBAT, PBT, PCT, PETG, PEIT, PET, plus préférentiellement le PET.

4. Procédé selon l'une quelconque des revendications précédentes, **caractérisé en ce que** la quantité de polyester d'intérêt engagée dans le réacteur est comprise entre 11% et 20% en poids par rapport au poids total du milieu réactionnel initial, préférentiellement 15%, +/- 2%.

5. Procédé selon l'une quelconque des revendications précédentes, **caractérisé en ce que** la quantité de polyester d'intérêt engagée dans le réacteur est supérieure ou égale à 15% en poids par rapport au poids total du milieu réactionnel initial, préférentiellement supérieure ou égale à 20% et/ou **en ce que** la concentration en AT dans la phase liquide du milieu réactionnel final est supérieure à 50kg/t, 60kg/t, 70kg/t, 80kg/t, 100kg/t ou 120kg/t.

6. Procédé selon l'une quelconque des revendications précédentes, **caractérisé en ce que** pendant l'étape de dépolymérisation le pH est régulé entre 6,5 et 8,5, préférentiellement entre 7,5 et 8,5, et/ou **caractérisé en ce que** la température est régulée entre 60°C et 80°C.

7. Procédé selon l'une quelconque des revendications précédentes, **caractérisé en ce que** l'étape de dépolymérisation dure au plus 150h, préférentiellement entre 1h et 120h, entre 1h et 100h, entre 1h et 72h, entre 1h et 48h.

8. Procédé selon l'une quelconque des revendications précédentes, **caractérisé en ce que** l'étape de récupération des sels d'AT solubilisés comprend une étape de séparation de la phase liquide contenant les sels d'AT du reste du milieu réactionnel.

9. Procédé selon l'une quelconque des revendications précédentes, **caractérisé en ce que** le procédé comprend une étape supplémentaire de récupération de l'AT par précipitation de l'AT contenu dans les sels d'AT, préférentiellement par acidification du milieu réactionnel.

10. Procédé selon la revendication 8 ou 9, **caractérisé en ce que** la phase liquide contenant les sels d'AT issue de l'étape de séparation est soumise à une étape de concentration et/ou à une étape de purification préalablement à l'étape de précipitation de l'AT, préférentiellement à une étape de purification par soumission de la phase liquide à une ou plusieurs étapes sélectionnées parmi l'ultrafiltration, la décoloration sur charbon, le passage sur échangeurs d'ions et la chromatographie.

11. Procédé selon l'une des revendications précédentes **caractérisé en ce que** le polyester d'intérêt est du PET et l'enzyme est une cutinase apte à dépolymériser le PET, préférentiellement sélectionnée parmi les enzymes ayant une séquence en acides aminés présentant au moins 75% d'identité avec SEQ ID N°1.

12. Procédé selon l'une des revendications précédentes **caractérisé en ce que** le pH est régulé pendant l'étape de dépolymérisation par l'ajout dans le milieu réactionnel d'une solution basique concentrée à au moins 10% +/- 1%, en poids de base par rapport au poids total de la solution basique, préférentiellement au moins 15% +/- 1%, plus préférentiellement au moins 20% +/- 1%.

13. Procédé selon l'une des revendications précédentes **caractérisé en ce que** la quantité de polyester d'intérêt engagée dans le réacteur est comprise entre 15% et 25%, préférentiellement 20% +/-2%, **en ce que** le pH est régulé entre 7,5 et 8,5 pendant l'étape de dépolymérisation par l'ajout dans le milieu réactionnel d'une solution basique concentrée à au moins 15% +/- 1%, et **en ce que** la concentration en AT dans la phase liquide du milieu réactionnel final est supérieure à 100kg/t.

14. Procédé selon l'une des revendications précédentes **caractérisé en ce qu'**il est mis en œuvre dans un réacteur dont le volume est supérieur à 1000L.

15. Réacteur d'un volume d'au moins 1000 Litres dans lequel se déroule au moins une étape de dépolymérisation enzymatique d'un polyester d'intérêt comprenant au moins une unité d'AT, **caractérisé en ce que** la quantité de polyester engagée dans le réacteur est supérieure ou égale à 11% en poids par rapport au poids total du milieu réactionnel initial, et **en ce que** la concentration en AT dans la phase liquide du milieu réactionnel final est supérieure à 40kg/t.

16. Réacteur selon la revendication 15 **caractérisé en ce que** la quantité de polyester d'intérêt engagée dans le réacteur est comprise entre 15% et 25%, préférentiellement 20% +/-2%, **en ce que** le pH et la température sont régulés dans le réacteur entre 7,5 et 8,5 et entre 60°C et 80°C pendant l'étape de dépolymérisation, et **en ce que** la concentration en AT dans la phase liquide du milieu réactionnel final est supérieure à 100kg/t.

## Patentansprüche

1. Verfahren zur Herstellung von Terephthalsäure (TA) aus mindestens einem gewünschten Polyester, der mindestens eine TA-Einheit enthält, umfassend einen Schritt der enzymatischen Depolymerisation des Polyesters, bei dem dieser Polyester in einem Reaktor unter Rühren mit mindestens einem Enzym in Kontakt gebracht wird, das diesen Polyester depolymerisieren kann, und einen Schritt der Rückgewinnung von TA-Salzen in gelöster Form, **dadurch gekennzeichnet, dass** die im Reaktor verwendete Polyestermenge größer oder gleich 11 Gew.-%, bezogen auf das Gesamtgewicht des anfänglichen Reaktionsmediums, beträgt, dass der pH-Wert während des Depolymerisationsschritts zwischen 6,5 und 9 geregelt wird, dass die Temperatur während des Depolymerisationsschritts zwischen 35°C und 90°C geregelt wird, dass der Depolymerisationsschritt zwischen 1 h und 120 h dauert und dass die TA-Konzentration in der flüssigen Phase des endgültigen Reaktionsmediums größer als 40 kg/t ist.

2. Verfahren nach Anspruch 1, **dadurch gekennzeichnet, dass** das Gewichtsverhältnis der eingesetzten Enzymmenge zur eingesetzten Menge des gewünschten Polyesters zwischen 0,01/1000 und 3/1000, vorzugsweise zwischen 1/1000 und 2/1000, liegt und/oder dass der gewünschte Polyester in Pulverform vorliegt, vorzugsweise mit einer Partikelgröße von weniger als 2 mm, besonders bevorzugt weniger als 1 mm.

3. Verfahren nach Anspruch 1 oder 2, **dadurch gekennzeichnet, dass** der gewünschte Polyester ausgewählt ist aus PTT, PBAT, PBT, PCT, PETG, PEIT und PET, besonders bevorzugt PET.

4. Verfahren nach einem der vorhergehenden Ansprüche, **dadurch gekennzeichnet, dass** die im Reaktor eingesetzte Menge des gewünschten Polyesters zwischen 11 und 20 Gew.-%, bezogen auf das Gesamtgewicht des anfänglichen Reaktionsmediums, vorzugsweise 15 %, +/- 2 %, beträgt.

5. Verfahren nach einem der vorhergehenden Ansprüche, **dadurch gekennzeichnet, dass** die im Reaktor eingebrachte Menge des gewünschten Polyesters größer oder gleich 15 Gew.-%,, vorzugsweise größer oder gleich 20 %, bezogen auf das Gesamtgewicht des anfänglichen Reaktionsmediums beträgt, und/oder dass die TA-Konzentration in der flüssigen Phase des endgültigen Reaktionsmediums größer als 50 kg/t, 60 kg/t, 70 kg/t, 80 kg/t, 100 kg/t oder 120 kg/t ist.

6. Verfahren nach einem der vorhergehenden Ansprüche, **dadurch gekennzeichnet, dass** während des Depolymerisationsschritts der pH-Wert zwischen 6,5 und 8,5, vorzugsweise zwischen 7,5 und 8,5, geregelt wird und/oder **dadurch gekennzeichnet, dass** die Temperatur zwischen 60 °C und 80 °C geregelt wird.

7. Verfahren nach einem der vorhergehenden Ansprüche, **dadurch gekennzeichnet, dass** der Depolymerisationsschritt höchstens 150 Stunden, vorzugsweise zwischen 1 Stunde und 120 Stunden, zwischen 1 Stunde und 100 Stunden, zwischen 1 Stunde und 72 Stunden, zwischen 1 Stunde und 48 Stunden dauert.

8. Verfahren nach einem der vorhergehenden Ansprüche, **dadurch gekennzeichnet, dass** der Schritt der Rückgewinnung der gelösten TA-Salze einen Schritt der Trennung der die TA-Salze enthaltenden flüssigen Phase vom Rest des Reaktionsmediums umfasst.

9. Verfahren nach einem der vorhergehenden Ansprüche, **dadurch gekennzeichnet, dass** das Verfahren einen zusätzlichen Schritt der Rückgewinnung des TA durch Ausfällung des in den TA-Salzen enthaltenen TA, vorzugsweise durch Ansäuern des Reaktionsmediums, umfasst.

10. Verfahren nach Anspruch 8 oder 9, **dadurch gekennzeichnet, dass** die aus dem Trennschritt resultierende, die TA-Salze enthaltende Flüssigphase vor dem TA-Fällungsschritt einem Konzentrierungsschritt und/oder einem Reinigungsschritt unterzogen wird, vorzugsweise einem Reinigungsschritt, indem die Flüssigphase einem oder mehreren Schritten unterzogen wird, ausgewählt aus Ultrafiltration, Entfärbung auf Kohle, Passage durch Ionenaustauscher und Chromatographie.

11. Verfahren nach einem der vorhergehenden Ansprüche, **dadurch gekennzeichnet, dass** der gewünschte Polyester aus PET ist und das Enzym eine Cutinase ist, die in der Lage ist, das PET zu depolymerisieren, vorzugsweise ausgewählt aus den Enzymen mit einer Aminosäuresequenz, die mindestens 75 % Identität mit SEQ ID Nr. 1 aufweist.

12. Verfahren nach einem der vorhergehenden Ansprüche, **dadurch gekennzeichnet, dass** der pH-Wert während des Depolymerisationsschritts reguliert wird, indem dem Reaktionsmedium eine konzentrierte basische Lösung mit mindestens 10 Gew.-% +/- 1 Gew.-% Base, bezogen auf das Gesamtgewicht der basischen Lösung, vorzugsweise mindestens 15% +/- 1%, stärker bevorzugt mindestens 20% +/- 1% zugesetzt wird.

13. Verfahren nach einem der vorhergehenden Ansprüche, **dadurch gekennzeichnet, dass** die Menge des in den Reaktor eingebrachten gewünschten Polyesters zwischen 15% und 25%, vorzugsweise 20% ± 2%, beträgt, dass der pH-Wert während des Depolymerisationsschritts durch Zugabe einer konzentrierten basischen Lösung von mindestens 15% ± 1% zum Reaktionsmedium zwischen 7,5 und 8,5 geregelt wird und dass die TA-Konzentration in der flüssigen Phase des endgültigen Reaktionsmediums über 100 kg/t liegt.

14. Verfahren nach einem der vorhergehenden Ansprüche, **dadurch gekennzeichnet, dass** es in einem Reaktor mit einem Volumen von über 1000 1 durchgeführt wird.

15. Reaktor mit einem Volumen von mindestens 1000 Litern, in dem mindestens ein Schritt der enzymatischen Depolymerisation eines gewünschten Polyesters, der mindestens eine TA-Einheit umfasst, stattfindet, **dadurch gekennzeichnet, dass** die im Reaktor verwendete Polyestermenge größer oder gleich 11 Gew.-%, bezogen auf das Gesamtgewicht des anfänglichen Reaktionsmediums, beträgt und dass die TA-Konzentration in der flüssigen Phase des endgültigen Reaktionsmediums größer als 40 kg/t ist.

16. Reaktor nach Anspruch 15, **dadurch gekennzeichnet, dass** die im Reaktor verwendete Menge des gewünschten Polyesters zwischen 15% und 25%, vorzugsweise 20% +/- 2%, beträgt, dass der pH-Wert und die Temperatur im Reaktor während des Depolymerisationsschritts zwischen 7,5 und 8,5 und zwischen 60°C und 80°C geregelt werden und dass die TA-Konzentration in der flüssigen Phase des endgültigen Reaktionsmediums über 100 kg/t liegt.

## Claims

1. A method for producing terephthalic acid (TA) from at least one polyester of interest comprising at least one TA unit, comprising a step of enzymatic depolymerization of the polyester, according to which said polyester is contacted with at least one enzyme capable of depolymerizing said polyester in a stirred reactor and a step of recovering TA salts in solubilized form, **characterized in that** the amount of polyester used in the reactor is greater than or equal to 11% by weight relative to the total weight of the initial reaction medium, **in that** the pH is regulated between 6.5 and 9 during the depolymerization step, **in that** the temperature is regulated between 35°C and 90°C during the depolymerization step, **in that** the depolymerization step lasts between 1h and 120h, and **in that** the concentration of TA in the liquid phase of the final reaction medium is greater than 40kg/t.

2. The method according to claim 1, **characterized in that** the weight ratio of the amount of enzyme used to the amount of polyester of interest used is comprised between 0.01/1000 and 3/1000, preferably between 1/1000 and 2/1000, and/or **in that** the polyester of interest is in powder form, preferably with a particle size of less than 2 mm, more preferably less than 1 mm.

3. The method according to claim 1 or 2, **characterized in that** the polyester of interest is selected from PTT, PBAT, PBT, PCT, PETG, PEIT, PET, more preferably PET.

4. The method according to any one of the preceding claims, **characterized in that** the amount of polyester of interest used in the reactor is comprised between 11% and 20% by weight relative to the total weight of the initial reaction medium, preferably 15%, +/- 2%.

5. The method according to any one of the preceding claims, **characterized in that** the amount of polyester of interest used in the reactor is greater than or equal to 15% by weight relative to the total weight of the initial reaction medium, preferably greater than or equal to 20% and/or **in that** the concentration of TA in the liquid phase of the final reaction medium is greater than 50kg/t, 60kg/t, 70kg/t, 80kg/t, 100kg/t or 120kg/t.

6. The method according to any one of the preceding claims, **characterized in that** during the depolymerization step the pH is regulated between 6.5 and 8.5, preferably between 7.5 and 8.5, and/or **characterized in that** the temperature is regulated between 60°C and 80°C.

7. The method according to any one of the preceding claims, **characterized in that** the depolymerization step lasts at most 150 hours, preferably between 1 hour and 120 hours, between 1 hour and 100 hours, between 1 hour and 72 hours, between 1 hour and 48 hours.

8. The method according to any one of the preceding claims, **characterized in that** the step of recovering the solubilized TA salts comprises a step of separating the liquid phase containing the TA salts from the rest of the reaction medium.

9. The method according to any one of the preceding claims, **characterized in that** the method comprises an additional step of recovering the TA by precipitation of the TA contained in the TA salts, preferably by acidification of the reaction medium.

10. The method according to claim 8 or 9, **characterized in that** the liquid phase containing the TA salts resulting from the separation step is subjected to a concentration step and/or to a purification step prior to the TA precipitation step, preferably to a purification step by subjecting the liquid phase to one or more steps selected from ultrafiltration, decolorization on charcoal, passage through ion exchangers and chromatography.

11. The method according to one of the preceding claims, **characterized in that** the polyester of interest is PET and the enzyme is a cutinase capable of depolymerizing PET, preferentially selected from enzymes having an amino acid sequence having at least 75% identity with SEQ ID N°1.

12. The method according to one of the preceding claims, **characterized in that** the pH is regulated during the depolymerization step by adding to the reaction medium a concentrated basic solution of at least 10% +/- 1%, by weight of base relative to the total weight of the basic solution, preferably at least 15% +/- 1%, more preferably at least 20% +/- 1%.

13. The method according to one of the preceding claims, **characterized in that** the amount of polyester of interest used in the reactor is comprised between 15% and 25%, preferably 20% +/- 2%, **in that** the pH is regulated between 7.5 and 8.5 during the depolymerization step by adding to the reaction medium a basic solution concentrated at least at 15% +/- 1%, and **in that** the concentration of TA in the liquid phase of the final reaction medium is greater than 100 kg/t.

14. The method according to one of the preceding claims, **characterized in that** it is implemented in a reactor whose volume is greater than 1000L.

15. A reactor with a volume of at least 1000 liters wherein at least one step of enzymatic depolymerization of a polyester of interest comprising at least one TA unit takes place, **characterized in that** the amount of polyester used in the reactor is greater than or equal to 11% by weight relative to the total weight of the initial reaction medium, and **in that** the TA concentration in the liquid phase of the final reaction medium is greater than 40 kg/t.

16. The reactor according to claim 15 **characterized in that** the amount of polyester of interest used in the reactor is comprised between 15% and 25%, preferably 20% +/-2%, **in that** the pH and the temperature are regulated in the reactor between 7.5 and 8.5 and between 60°C and 80°C during the depolymerization step, and **in that** the concentration of TA in the liquid phase of the final reaction medium is greater than 100 kg/t.
